# EUROPEAN PATENT APPLICATION

(11) **EP 1 897 872 A1**
(43) Date of publication of application: **12.03.2008**
(21) Application number: 06756770.1
(22) Date of filing: 24.05.2006
(51) Int. Cl.: C07D 277/28, A61K 31/496, A61P 3/06, A61P 43/00

(54) **ACTIVATOR FOR PEROXISOME PROLIFERATOR-ACTIVATED RECEPTOR**

(30) Priority: 25.05.2005 JP 2005152949
(71) Applicant: Nippon Chemiphar Co., Ltd., Chiyoda-ku Tokyo 1010032 (JP)
(72) Inventor: SAKUMA, Shogo, 3420041 (JP); MOCHIDUKI, Nobutaka, 2701166 (JP); TAKAHASHI, Rie, 3410005 (JP); HIRAI, Toshitake, 2780036 (JP); YAMAKAWA, Tomio, 2770884 (JP); MASUI, Seiichiro, 3620072 (JP)
(74) Representative: Jones Day
(86) International application number: PCT/JP2006/310822
(87) International publication number: WO 2006/126714

(57) **Abstract**

A compound having the following formula or its salt is used as an activator for PPAR: 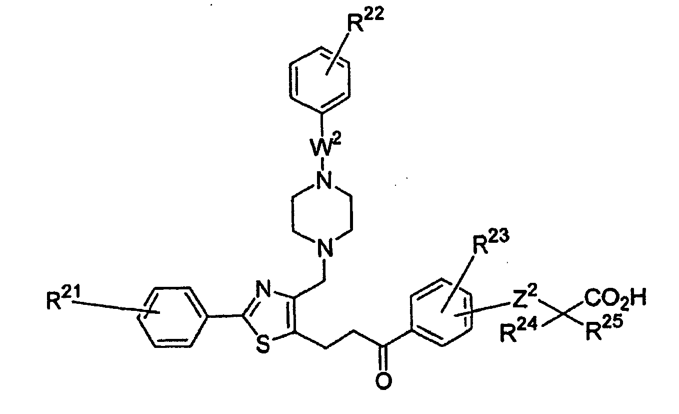 [in which W² represents a bond, C(C=O) or CH₂; Z² represents an oxygen or sulfur atom; R²¹, R²² and R²³ independently represent a hydrogen atom, an alkyl group having 1 to 8 carbon atoms, an alkoxy group having 1 to 8 carbon atoms, a halogen atom, an alkyl group having 1 to 8 carbon atoms which is substituted with a halogen atom or the like; and R²⁴ and R²⁵ independently represent a hydrogen atom, an alkyl group having 1 to 8 carbon atoms or an alkyl group having 1 to 8 carbon atoms which is substituted with a halogen atom].

## Description

### [FIELD OF THE INVENTION]

The present invention relates to an activator for peroxisome proliferator-activated receptor(PPAR).

### [BACKGROUND OF THE INVENTION]

As for the peroxisome proliferator-activated receptor (PPAR), it is known that there are three subtypes such as PPARα, PPARγ and PPARδ. --- Proc. Natl. Acad. Sci. USA, 91, p7335-7359, 1994 (Non-Patent Publication 1).

Until now, a transcription-activating function, a glycemic index-depressing function, a lipid metabolism-improving function and the like have been reported on each subtype of PPAR for various compounds.

For instance, it has been reported that GW-590735 (GSK), KRP-101 (Kyorin), and NS-220 (Roche-Nihon Shinyaku) has a function as a selective α-agonist having a lipid metabolism-improving function. --- J. Pharmacol. Exp. Ther., 309(3):970, Jun. 2001 (Non-Patent Publication 2).

Further, various pharmacologically active compounds having dual agonist functions for PPARγ and PPARα are known. For example, there are reports on KRP-297 (Merck-Kyorin) belonging to TZD (thiazolidindione) derivatives, Muraglitazar (BMS/Merck) belonging to non-TZD derivatives, and Tesaglitazar (AstraZeneca). These compounds are represented by the following formulas:

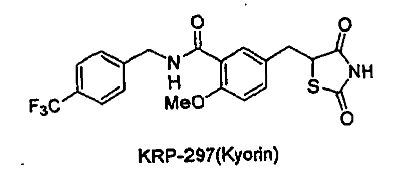

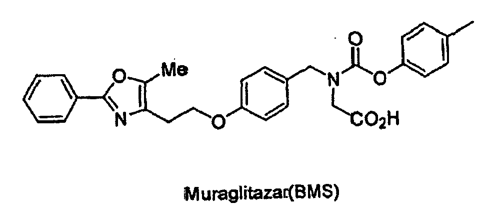

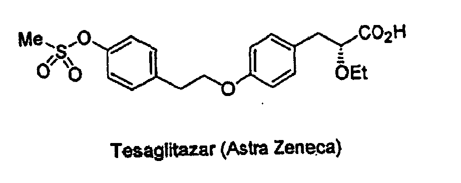

Further, GW-501516 (GSK) having the following formula is known as a PPARδ selective agonist: 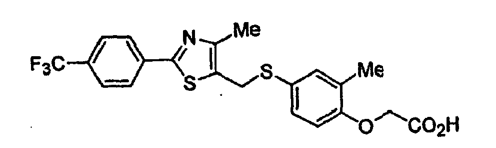 Recently, it has been reported that the above-mentioned compound has been studied for the use as a lipid metabolism-improving agent. --- WO 01/603 (Patent Publication 1).

Furthermore, it has been reported that a piperazine derivative (which is a fibrate derivative derived from GW-501516 by introducing a phenylpiperazine residue into methyl of the thiazole ring) has a strong glycemic index-depressing function --- WO 02/59098 (Patent Publication 2), WO 02/67912 (Patent Publication 3):

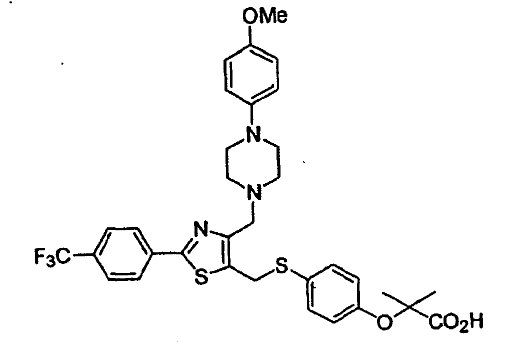

The present inventors already filed patent applications for compounds having a thiazole ring which have a transcription-activating function for PPARδ. --- WO 02/14291 (Patent Publication 4), WO 03/16291 (Patent Publication 5), etc.

The compounds of the present invention which are represented by the below-illustrated formulas (I) and (II) are apparently different in structure from the aforementioned GW-501516 and the like and are not described in any of the aforementioned publications.

### [DISCLOSURE OF THE INVENTION]

The invention has an object to provide compounds having the following formula (I) or (II), which have an activating function for peroxisome proliferator-activated receptor.

In one aspect, the invention resides in compounds having the following formula (I) or salts thereof: 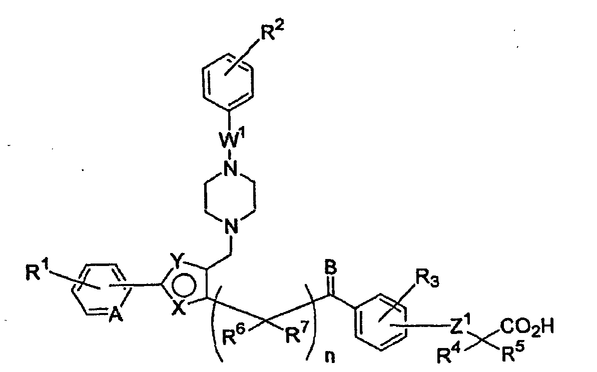 in which
A represents CH or a nitrogen atom;
B represents an oxygen atom or C(R⁸) (R⁹) in which each of R⁸ and R⁹ independently represents a hydrogen atom or an alkyl group having 1 to 8 carbon atoms;
W¹ represents a bond, C (=O), or (-C(R¹⁰) (R¹¹)-)ₘ in which each of R¹⁰ and R¹¹ independently a hydrogen or an alkyl group having 1 to 8 carbon atoms and m is an integer of 1 to 3;
X and Y differ from each other, and each represents an oxygen atom, a sulfur atom, a nitrogen atom, or CR¹² in which R¹² represents a hydrogen atom or an alkyl group having 1 to 8 carbon atoms;
Z¹ represents a bond, an oxygen atom, a sulfur atom, or C(R¹³) (R¹⁴) in which each of R¹³ and R¹⁴ independently represents a hydrogen atom or an alkyl group having 1 to 8 carbon atoms;
each of R¹, R² and R³ independently represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms which is substituted with a halogen atom, an alkoxy group having 1 to 8 carbon atoms which is substituted with a halogen atom, hydroxyl, nitro, an acyl group having 2 to 8 carbon atoms, an aryl group having 6 to 10 carbon atoms, or a 5- or 6-membered heterocyclic group;
each of R⁴ and R⁵ independently represents a hydrogen atom, an alkyl group having 1 to 8 carbon atoms, or an alkyl group having 1 to 8 carbon atoms which is substituted with a halogen atom;
each of R⁶ and R⁷ independently represents a hydrogen atom, an alkyl group having 1 to 8 carbon atoms, an alkenyl group having 2 to 8 carbon atoms, an alkynyl group having 2 to 8 carbon atoms, or an alkyl group having 1 to 8 carbon atoms which is substituted with a halogen atom; and
n represents an integer of 1 to 5.

In another aspect, the invention resides in compounds having the following formula (II) or salts thereof: 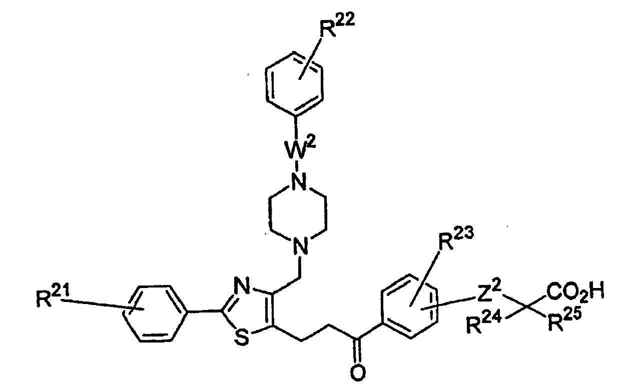 in which
W² represents a bond, C(=0), or -CH₂;
Z² represents an oxygen atom or a sulfur atom;
each of R²¹, R²² and R²³ independently represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms which is substituted with a halogen atoms, an alkoxy group having 1 to 8 carbon atoms which is substituted with a halogen atom, hydroxyl, nitro, an acyl group having 2 to 8 carbon atoms, an aryl group having 6 to 10 carbon atoms, or a 5- or 6-membered heterocyclic group;
each of R²⁴ and R²⁵ independently represents a hydrogen atom, an alkyl group having 1 to 8 carbon atoms, or an alkyl group having 1 to 8 carbon atoms which is substituted with a halogen atom.

In still another aspect, the invention resides in an activator for peroxisome proliferator-activated receptor containing a compound of the formulas (I) or (II) as an effective component.

### [PREFERRED EMBODIMENTS OF THE INVENTION]

The invention is described below in detail.

In the formula (I), examples of the alkyl groups having 1 to 8 carbon atoms for R¹, R², R³, R⁴, R⁵, R⁶, R⁷, R⁸, R⁹, R¹⁰, R¹¹, R¹², R¹³ and R¹⁴ include methyl, ethyl, propyl, isopropyl, butyl, i-butyl, t-butyl, pentyl and hexyl.

Examples of the alkenyl groups having 2 to 8 carbon atoms for R¹, R², R³, R⁶ and R⁷ include vinyl and allyl.

Examples of the alkynyl groups having 2 to 8 carbon atoms for R¹, R², R³, R⁶ and R⁷ include propargyl.

Examples of the alkoxy groups having 1 to 8 carbon atoms for R¹, R², and R³ include methoxy, ethoxy, propoxy, isopropoxy, butoxy, i-butoxy, t-butoxy, pentyloxy and hexyloxy.

Examples of the halogen atoms for R¹, R², and R³ include fluorine, chlorine, and bromine.

Examples of the alkyl groups having 1 to 8 carbon atoms which are substituted with a halogen atom for R¹, R², R³, R⁴, R⁵, R⁶, and R⁷ include methyl, ethyl, propyl, isopropyl, butyl, and t-butyl which are substituted with 1 to 3 halogen atoms such as fluorine, chlorine, and bromine. Preferred are trifluoromethyl, chloromethyl, 2-chloroethyl, 2-bromoethyl, and 2-flouroethyl.

Examples of the alkoxy groups having 1 to 8 carbon atoms which are substituted with a halogen atom for R¹, R², and R³ include methoxy, ethoxy, propoxy, isopropoxy, butoxy, and t-butoxy which are substituted with 1 to 3 halogen atoms such as fluorine, chlorine, and bromine. Preferred are trifluoromethyloxy, chloromethyloxy, 2-chloroethyloxy, 2-bromoethyloxy, and 2-flouroethyloxy.

Examples of the acyl groups having 2 to 8 carbon atoms for R¹, R² and R³ include acetyl and propionyl.

Examples of the aryl groups having 6 to 10 carbon atoms for R¹, R² and R³ include phenyl.

Examples of the 5- or 6-membered heterocyclic groups for R¹, R² and R³ include pyridyl.

In the formula (II), the alkyl groups having 1 to 8 carbon atoms, alkenyl groups having 2 to 8 carbon atoms, alkynyl groups having 2 to 8 carbon atoms, alkoxy groups having 1 to 8 carbon atoms, halogen atoms, alkyl groups having 1 to 8 carbon atoms which are substituted with a halogen atom, alkoxy groups having 1 to 8 carbon atoms which are substituted with a halogen atom, hydroxyls, nitros, acyl groups having 2 to 8 carbon atoms, aryl groups having 6 to 10 carbon atoms, and 5- or 6-membered heterocyclic groups for R²¹, R²² and R²³ can be those described for R¹, R² and R³ in the formula (I).

In the formula (II), the alkyl groups having 1 to 8 carbon atoms and alkyl groups having 1 to 8 carbon atoms which are substituted with a halogen atom for R²⁴ and R²⁵ can be those described for R⁴ and R⁵ in the formula (I).

It should be noted that R¹, R² and R³ in the formula (I) and R²¹, R²² and R²³ in the formula (II) can be attached to the benzene ring or the like in numbers of 1 to 3 in which the same or different groups can be attached to the same ring.

Examples of the preferred compounds of the invention are set forth below.
(1) The compounds of the formula (I) and their salts in which A is CH are preferred.
(2) The compounds of the formula (I), the compounds of (1) above, and their salts in which B is an oxygen atom are preferred.
(3) The compounds of the formula (I), the compounds of (1) or (2) above, and their salts in which W¹ is a bond are preferred.
(4) The compounds of the formula (I), the compounds of (1) or (2) above, and their salts in which W¹ is methylene or C(=O) are preferred.
(5) The compounds of the formula (I), the compounds of (1) to (4) above, and their salts in which X and Y are different from each other and each is an oxygen atom, a sulfur atom or a nitrogen atom are preferred.
(6) The compounds of the formula (I), the compounds of (1) to (4) above, and their salts in which X is a sulfur atom and Y is a nitrogen atom are preferred.
(7) The compounds of the formula (I), the compounds of (1) to (6) above, and their salts in which Z¹ is an oxygen atom or a sulfur atom are preferred.
(8) The compounds of the formula (I), the compounds of (1) to (7) above, and their salts in which R¹, R² and R³ independently is a hydrogen atom, an alkyl group having 1 to 8 carbon atoms, an alkenyl group having 2 to 8 carbon atoms, an alkoxy group having 1 to 8 carbon atoms, a halogen atom, an alkyl group having 1 to 8 carbon atoms which is substituted with a halogen atom, or an alkoxy group having 1 to 8 carbon atoms which is substituted with a halogen atom are preferred.
(9) The compounds of the formula (I), the compounds of (1) to (8) above, and their salts in which each of R⁴ and R⁵ independently is a hydrogen atom or methyl are preferred.
(10) The compounds of the formula (I), the compounds of (1) to (9) above, and their salts in which each of R⁶ and R⁷ independently is a hydrogen atom or an alkyl group having 1 to 8 carbon atoms are preferred.
(11) The compounds of the formula (I), the compounds of (1) to (10) above, and their salts in which n is an integer of 2 to 4 are preferred.
(12) The compounds of the formula (I), the compounds of (1) to (10) above, and their salts in which n is 2 are preferred.
(13) The compounds of the formula (II) and their salts in which W² is a bond are preferred.
(14) The compounds of the formula (II), the compounds of (13) above, and their salts in which R²¹, R²² and R²³ independently is a hydrogen atom, an alkyl group having 1 to 8 carbon atoms, an alkenyl group having 2 to 8 carbon atoms, an alkoxy group having 1 to 8 carbon atoms, a halogen atom, an alkyl group having 1 to 8 carbon atoms which is substituted with a halogen atom, or an alkoxy group having 1 to 8 carbon atoms which is substituted with a halogen atom are preferred.
(15) The compounds of the formula (II), the compounds of (13) or (14) above, and their salts in which each of R²⁴ and R²⁵ independently is a hydrogen atom or methyl are preferred.

The compounds of the invention which are represented by the formula (I) or (II) can be in the form of a pharmacologically acceptable salts such as alkali metal salts, e.g., salts of sodium, potassium and lithium.

The compounds of the invention can be in the optically active forms, and in the form of optical isomers such as compounds of a racemic form or geometric isomers such as compounds of a cis- or trans form.

The schemes for synthesis of the compounds of the invention having the formula (I) are illustrated below.

### [Compounds of the formula (I) in which Z¹ is an oxygen atom]

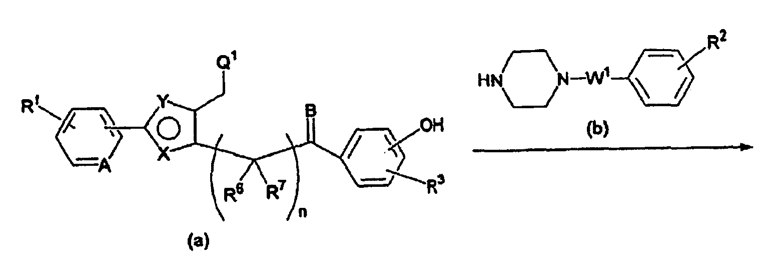

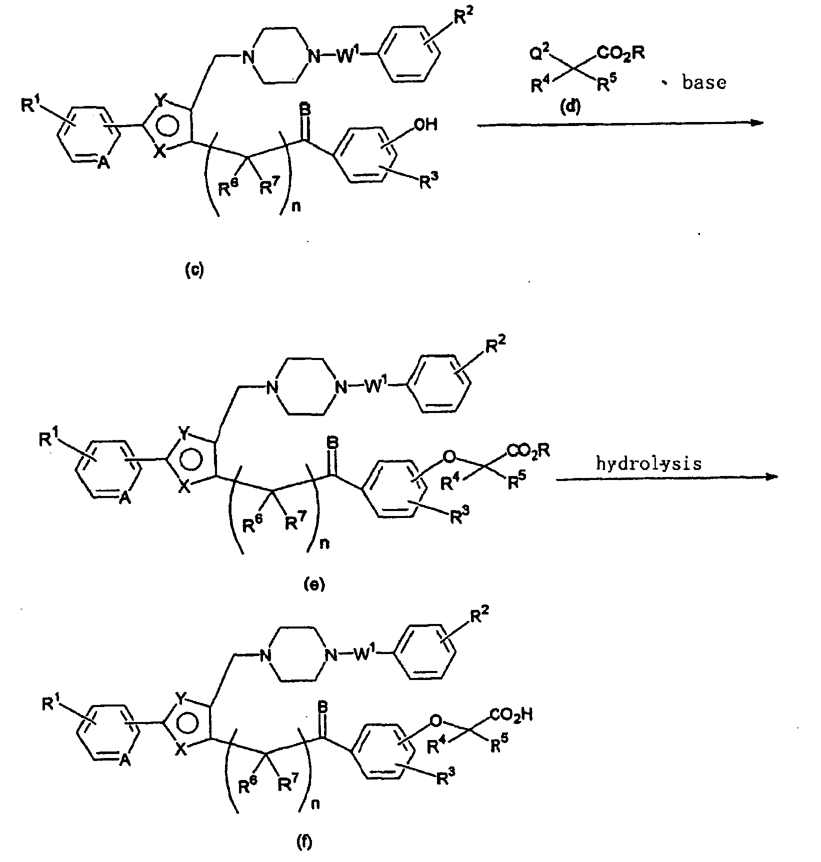

In the above-illustrated formulas, each of Q¹ and Q² represents a halogen atom such as chlorine, R represents a lower alkyl group such as ethyl, and A, B, X, Y, W¹, R¹, R², R³, R⁴, R⁵, R⁶ and R⁷ are the same as those described hereinbefore.

The compound of the formula (c) can be obtained by reacting a compound of the formula (a) and a compound of the formula (b) in a solvent such as THF which is inert in the reaction.

Then, a compound of the formula (c) is reacted with a compound of the formula (d) in a solvent such as acetone or 2-butanone which is inert in the reaction in the presence of potassium carbonate, to give a compound of the formula (e).

The compound of the invention represented by the formula (f) can be obtained by subjecting a compound of the formula (e) to hydrolysis using lithium hydroxide or the like.

A compound of the formula (a) which is the starting compound can be obtained in the below-illustrated manner.

### [Synthesis of Starting Compound (1)]

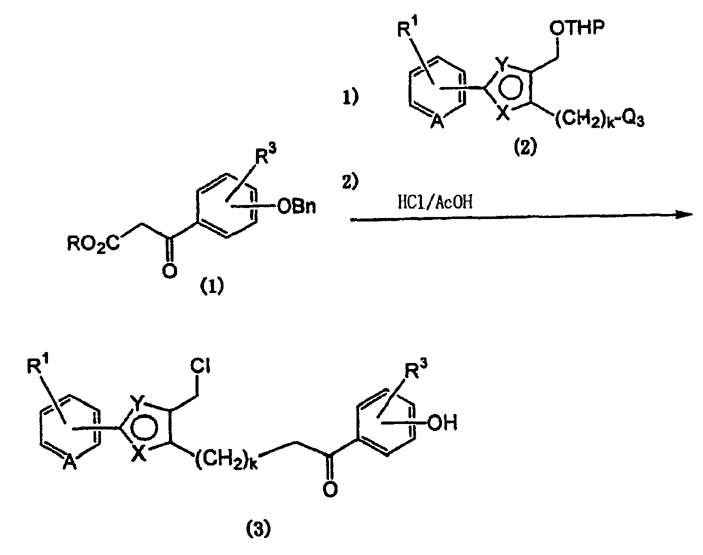

In the formulas, R represents a lower alkyl group such as ethyl, Bn represents benzyl, THP represents tetrahydropyranyl, J represents an integer of 1 to 4, Q³ represents a halogen atom such as chlorine, and A, X, Y, R¹ and R³ are the same as those described hereinbefore.

A compound of the formula (3) can be obtained by reacting a benzyl acetate ester of the formula (1) and a compound of the formula (b) in a solvent such as THF which is inert in the reaction in the presence of a base such as sodium hydroxide, and then subjecting the resulting compound to decarbonation, benzyl-elimination, and chlorine-replacement of O-THP using hydrochloric acid/acetic acid.

### [Synthesis of Starting Compound (2)]

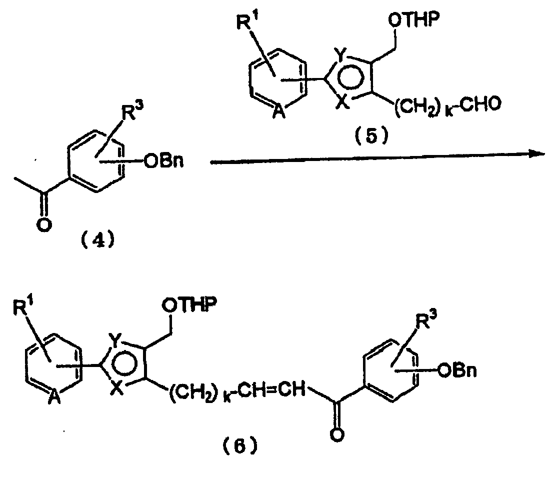

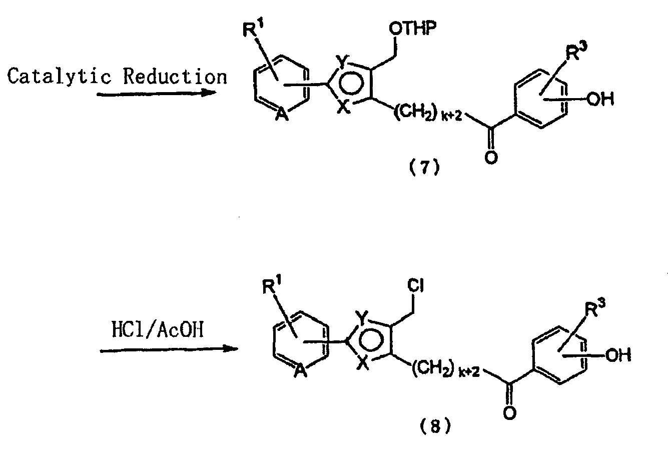

In the formulas, Bn represent benzyl, THP represents tetrahydropyranyl, k represent an integer of 1 to 3, and A, X, Y, R¹ and R³ are the same as those described hereinbefore.

A vinyl compound of the formula (6) can be obtained by subjecting an aldehyde compound of the formula (5) and an acetophenone compound of the formula (4) to an aldol condensation reaction in the presence of a base. A phenol compound of the formula (7) can be obtained by subjecting the vinyl compound of the formula (6) to reactions of reduction of its olefinic moiety and benzyl-elimination. Thus obtained compound of the formula (7) can be subjected to a reaction with hydrochloric acid/acetic acid to give a compound of the formula (8).

The compounds (1) or (II) of the invention can be prepared by the aforementioned synthesis processes, the below-mentioned working examples, and with reference to the aforementioned patent publications and other publications.

Examples of the compounds of the invention are set forth in the following tables.
(1) Compounds represented by the following formula: 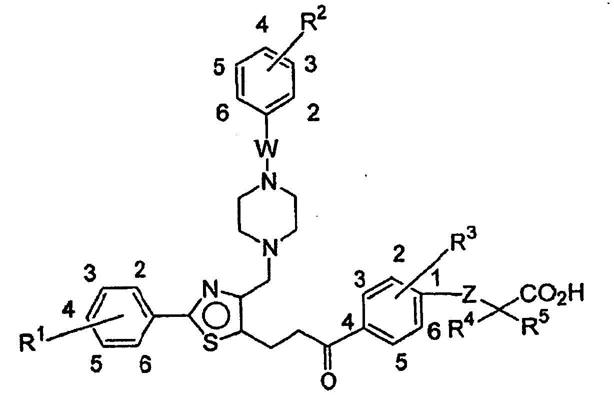 in which each of W, Z, R¹, R², R³, R⁴, and R⁵ is that set forth in Tables 1 to 3.

**Table 1**

| W | Z | R¹ | R² | R³ | R⁴/R⁵ |
|---|---|---|---|---|---|
| bond | O | 4-CF₃ | 4-OMe | 2-Me | Me/H |
| bond | O | 4-CF₃ | 4-OMe | 2-Me | H/H |
| bond | O | 4-CF₃ | 4-OMe | 2-Me | Me/Me |
| bond | O | 4-CF₃ | 3-OMe | 2-Me | Me/Me |
| bond | O | 4-CF₃ | 4-CF₃ | 2-Me | Me/Me |
| bond | O | 4-CF₃ | 4-Me | 2-Me | Me/Me |
| CH₂ | O | 4-CF₃ | 4-OMe | 2-Me | Me/Me |
| C=O | O | 4-CF₃ | 4-OMe | 2-Me | Me/Me |
| bond | O | 4-CF₃ | 2-OMe | 2-Me | Me/Me |

**Table 2**

| W | Z | R¹ | R² | R³ | R⁴/R⁵ |
|---|---|---|---|---|---|
| bond | O | 4-CF₃ | 4-F | 2-Me | Me/Me |
| bond | O | 4-CF₃ | 4-iPr | 2-Me | Me/Me |
| bond | O | 4-CF₃ | 3,4-OMe | 2-Me | Me/Me |
| bond | O | 4-CF₃ | 4-OEt | 2-Me | Me/Me |
| bond | O | 4-CF₃ | 4-OMe | H | Me/Me |
| bond | O | 4-CF₃ | 4-OCF₃ | 2-Me | Me/Me |
| bond | O | 4-CF₃ | 4-OiPr | 2-Me | Me/Me |
| bond | O | 4-Me | 4-OMe | 2-Me | Me/Me |
| bond | O | 4-OCF₃ | 4-OMe | 2-Me | Me/Me |

**Table 3**

| W | Z | R¹ | R² | R³ | R⁴/R⁵ |
|---|---|---|---|---|---|
| bond | O | 4-OMe | 4-OMe | 3-Me | Me/Me |
| bond | O | 4-Cl | 4-OMe | 3-Me | Me/Me |
| bond | O | 4-CF₃ | 4-OMe | 2-allyl | Me/Me |
| bond | S | 4-CF₃ | 4-OMe | 2,3-Me | Me/Me |
| bond | S | 4-CF₃ | 4-CF₃ | 3,6-Me | Me/Me |
| bond | S | 4-CF₃ | 4-iPr | 2-Et | Me/Me |
| C=O | S | 4-Me | 4-OMe | 2-Cl | Me/Me |
| C=O | S | 4-Me | 4-OiPr | 2-Me | Me/H |

(2) Compounds represented by the following formula: 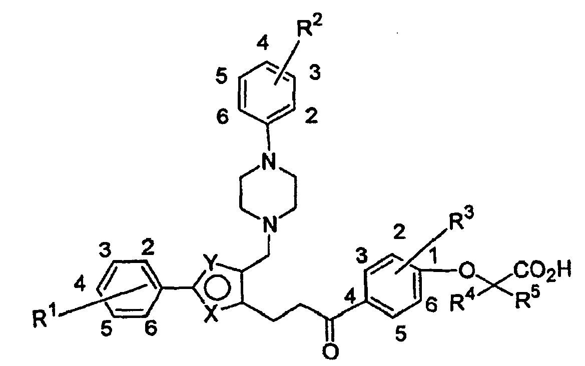 in which each of X, Y, R¹, R², R³, R⁴, and R⁵ is that set forth in Tables 4 and 6.

**Table 4**

| X | Y | R¹ | R² | R³ | R⁴/R⁵ |
|---|---|---|---|---|---|
| O | N | 4-CF₃ | 4-OMe | 2-Me | Me/Me |
| N | O | 4-Me | 4-OMe | 2-allyl | Me/H |
| N | S | 4-OCF₃ | 4-OMe | 2-OMe | H/H |
| CH | S | 4-Cl | 4-OMe | 2-Cl | Me/Me |
| S | CH | 4-CF₃ | 4-OMe | 2-Me | Me/Me |
| O | N | 4-CF₃ | 4-CF₃ | 2,6-Me | Me/Me |
| N | O | 4-Me | 4-CF₃ | 2-allyl | Me/H |
| N | S | 4-OCF₃ | 4-CF₃ | 2-OMe | H/H |
| CH | S | 4-Cl | 4-CF₃ | 2-Cl | Me/Me |

**Table 5**

| X | Y | R¹ | R² | R³ | R⁴/R⁵ |
|---|---|---|---|---|---|
| S | CH | 4-CF₃ | 4-CF₃ | 2-Me | Me/Me |
| O | N | 4-CF₃ | 4-iPr | 2-Me | Me/Me |
| N | O | 2,4-Cl | 4-iPr | 2-allyl | H/H |
| N | S | 4-OCF₃ | 4-iPr | 2-allyl | Me/Me |
| CH | S | 4-Cl | 4-iPr | 3-Cl | Me/Me |
| S | CH | 4-CF₃ | 4-iPr | 2-Me | Me/Me |
| O | N | 4-CF₃ | 3-OMe | 2-Me | H/H |
| N | O | 4-Me | 3-OMe | 2-allyl | Me/Me |
| N | S | 4-OCF₃ | 3-OMe | 3-OMe | Me/Me |

**Table 6**

| X | Y | R¹ | R² | R³ | R⁴/R⁵ |
|---|---|---|---|---|---|
| CH | S | 4-Cl | 3-OMe | 2-Cl | Me/Me |
| S | CH | 4-CF₃ | 3-OMe | 2-Me | H/H |
| O | N | 4-CF₃ | 4-Cl | 2-Me | Me/Me |
| N | O | 2-OH,4-Cl | 4-Cl | 2-allyl | Me/Me |
| N | S | 4-OCF₃ | 4-Cl | 2-OMe | Me/H |
| CH | S | 4-Cl | 4-Cl | 2-Cl | Me/Me |
| S | CH | 4-CF₃ | 4-Cl | 2-Me | Me/Me |

(3) Compounds represented by the following formula: 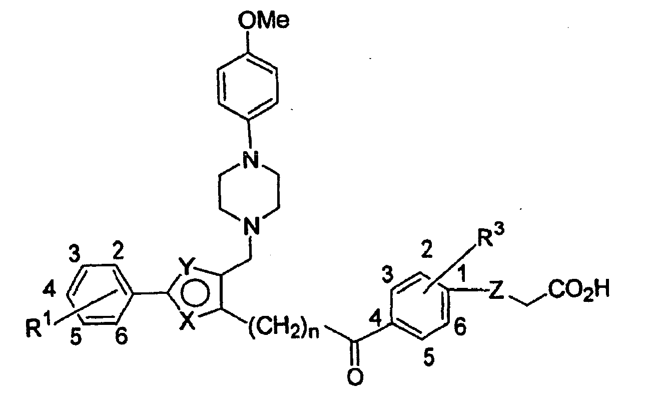 in which each of X, Y, Z, R¹, R³, and n is that set forth in Tables 7 and 10.

**Table 7**

| X | Y | Z | R¹ | R³ | n |
|---|---|---|---|---|---|
| S | N | bond | 4-CF₃ | H | 2 |
| S | N | bond | 4-OCF₃ | H | 3 |
| S | N | bond | 4-Me | H | 4 |
| S | CH | bond | 4-OMe | H | 2 |
| S | CH | bond | 2,4-Cl | H | 3 |
| S | CH | bond | 4-CF₃ | H | 4 |
| O | N | bond | 4-OCF₃ | H | 2 |
| O | N | bond | 4-Me | H | 3 |

**Table 8**

| X | Y | Z | R¹ | R³ | n |
|---|---|---|---|---|---|
| O | N | bond | 4-OMe | H | 4 |
| S | N | CH₂ | 4-Cl | H | 2 |
| S | N | CH₂ | 4-CF₃ | H | 3 |
| S | N | CH₂ | 4-OCF₃ | H | 4 |
| S | CH | CH₂ | 4-Me | H | 2 |
| S | CH | CH₂ | 4-OMe | H | 3 |
| S | CH | CH₂ | 4-Cl | H | 4 |
| S | N | bond | 4-CF₃ | 2-Me | 2 |

**Table 9**

| X | Y | Z | R¹ | R³ | n |
|---|---|---|---|---|---|
| S | N | bond | 4-OCF₃ | 3-Me | 3 |
| S | N | bond | 4-Me | 2-Cl | 4 |
| S | CH | bond | 4-OMe | 2-F | 2 |
| S | CH | bond | 4-Cl | 2,3-Me | 3 |
| S | CH | bond | 4-CF₃ | 2-Ome | 4 |
| O | N | bond | 4-OCF₃ | 3-Ome | 2 |
| O | N | bond | 4-Me | 2,6-OMe | 3 |
| O | N | bond | 4-OMe | 3,5-OMe | 4 |

**Table 10**

| X | Y | Z | R¹ | R³ | n |
|---|---|---|---|---|---|
| S | N | CH₂ | 2,4-Cl | 2-Me | 2 |
| S | N | CH₂ | 4-CF₃ | 3-Me | 3 |
| S | N | CH₂ | 4-OCF₃ | 2-Cl | 4 |
| S | CH | CH₂ | 4-Me | 2-F | 2 |
| S | CH | CH₂ | 4-OMe | 2,3-Me | 3 |
| S | CH | CH₂ | 4-Cl | 2-OMe | 4 |

(4) Compounds represented by the following formula: 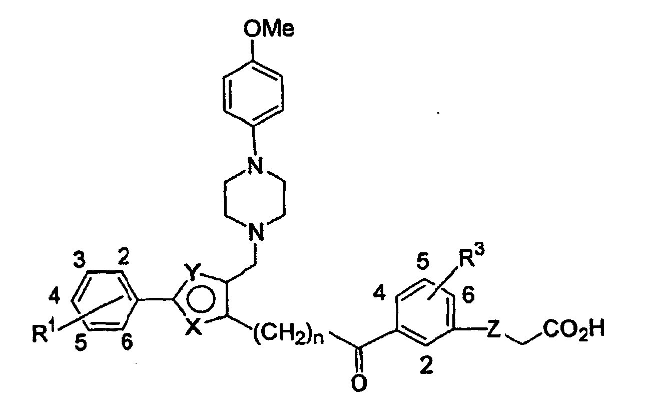 in which each of X, Y, Z, R¹, R³, and n is that set forth in Tables 11 and 14.

**Table 11**

| X | Y | Z | R¹ | R³ | n |
|---|---|---|---|---|---|
| S | N | bond | 4-CF₃ | H | 2 |
| S | N | bond | 4-OCF₃ | H | 3 |
| S | N | bond | 4-Me | H | 4 |
| S | CH | bond | 4-OMe | H | 2 |
| S | CH | bond | 2,4-Cl | H | 3 |
| S | CH | bond | 4-CF₃ | H | 4 |
| O | N | bond | 4-OCF₃ | H | 2 |
| O | N | bond | 4-Me | H | 3 |

**Table 12**

| X | Y | Z | R¹ | R³ | n |
|---|---|---|---|---|---|
| O | N | bond | 4-OMe | H | 4 |
| S | N | CH₂ | 2,4-Cl | H | 2 |
| S | N | CH₂ | 4-CF₃ | H | 3 |
| S | N | CH₂ | 4-OCF₃ | H | 4 |
| S | CH | CH₂ | 3,4-Me | H | 2 |
| S | CH | CH₂ | 4-OMe | H | 3 |
| S | CH | CH₂ | 4-Cl | H | 4 |
| S | N | bond | 4-CF₃ | 2-Me | 2 |

**Table 13**

| X | Y | Z | R¹ | R³ | n |
|---|---|---|---|---|---|
| S | N | bond | 4-OCF₃ | 6-Me | 3 |
| S | N | bond | 4-Me | 2-Cl | 4 |
| S | CH | bond | 4-OMe | 2-F | 2 |
| S | CH | bond | 2-OH,4-Cl | 2,5-Me | 3 |
| S | CH | bond | 4-CF₃ | 2-Ome | 4 |
| O | N | bond | 4-OCF₃ | 4-OMe | 2 |
| O | N | bond | 4-Me | 5-Ome | 3 |
| O | N | bond | 4-OMe | 6-OMe | 4 |

**Table 14**

| X | Y | Z | R¹ | R³ | n |
|---|---|---|---|---|---|
| S | N | CH₂ | 4-Cl | 2-Me | 2 |
| S | N | CH₂ | 4-CF₃ | 6-allyl | 3 |
| S | N | CH₂ | 4-OCF₃ | 2-Cl | 4 |
| S | CH | CH₂ | 4-Me | 2-F | 2 |
| S | CH | CH₂ | 4-OMe | 2,4-Me | 3 |
| S | CH | CH₂ | 4-Cl | 2-OMe | 4 |

(5) Compounds represented by the following formula: 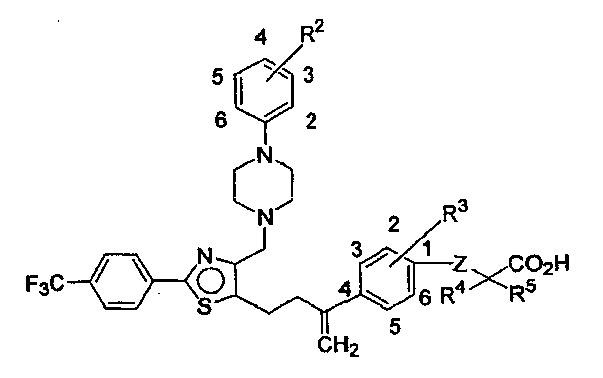 in which each of W, Z, R², R³, R⁴, and R⁵ is that set forth in Tables 15 and 16.

**Table 15**

| W | Z | R² | R³ | R⁴/R⁵ |
|---|---|---|---|---|
| bond | O | 4-OMe | 2-Me | Me/H |
| bond | O | 4-OMe | 2-Cl | H/H |
| bond | O | 4-OMe | 2-Me | Me/Me |
| bond | O | 3-OMe | 2-allyl | Me/Me |
| bond | O | 4-CF₃ | 2-Me | Me/Me |
| bond | O | 4-Me | 2-Me | Me/Me |
| CH₂ | O | 4-OMe | 2-Me | Me/Me |
| C=O | O | 4-OMe | 2-Me | Me/Me |

**Table 16**

| W | Z | R² | R³ | R⁴/R⁵ |
|---|---|---|---|---|
| bond | O | 2-OMe | 3-Me | Me/Me |
| bond | S | 4-F | 3,5-Me | Me/Me |
| bond | O | 4-iPr | 2-Me | Me/Me |
| bond | O | 3,4-OMe | 2-Me | Me/Me |
| bond | S | 4-OEt | 2-Me | Me/Me |
| bond | O | 4-OMe | H | Me/Me |
| bond | O | 4-OCF₃ | 2-Me | Me/Me |
| bond | O | 4-OiPr | 2-Me | Me/Me |

The pharmacological effects of the invention are described below.

For determining PPAR activating effects of the compounds according to the invention were measured by the following method:

A receptor expression plasmid (pSG5-GAL4-hPPAR α or γ or δ (LBD)), a luciferase expression plasmid (pUC8-MH100x4-TK-Luc) and β-galactosidase expression plasmid (pCMX-β-GAL)(Kliewer, S.A., et. al.,(1992) Nature, 358:771-774) are transfected into CV-1 cells (ATCC). After gene transfer utilizing a lipofection reagent DMRIE-C or Lipofectamin 2000 (Invitrogen), it is incubated for 42 hours in the presence of the test compound. Then, the luciferase activity and β-GAL activity are measured on the soluble cells. The luciferase activity is calibrated by the β-GAL activity. A relative ligand activity is calculated for each of the PPAR α, γ and δ under the following conditions, and EC₅₀ is obtained: a relative activity of PPAR α is calculated in consideration of a luciferase activity (assigned to 100%) of cells treated with GW-590735 (PPARα-selective agonist); a relative activity of PPAR γ is calculated in consideration of a luciferase activity (assigned to 100%) cells treated with Rosiglitazone; and a relative activity of PPAR δ is calculated in consideration of a luciferase activity (assigned to 100%) of cells treated with GW-501516. See the below-described Example 17.

As is apparent from Table 17, the compounds of the invention show excellent PPAR activating effect.

Since the compound of the invention having the formula (I) or (II) shows excellent PPAR activating effect, it is expected to serve as remedy for prevention and treatment of the following diseases: hyperglycemia, obesity, syndrome X, hyperchloresterolemia, hyperlipopreoteinemia, other dysbolismic diseases, hiperlipemia, arterial sclerosis, diseases of cardiovascular systems, hyperphagia, ischemic diseases, malignant tumors such as lung cancer, mammary cancer, colonic cancer, cancer of great intestine abd ovary cancer, Alzheimer's disease, and inflammatory disease.

The compound of the invention can be administered to human beings by ordinary administration methods such as oral administration or parenteral administration.

The compound can be granulated in ordinary manners for the preparation of pharmaceuticals. For instance, the compound can be processed to give pellets, granule, powder, capsule, suspension, injection, suppository, and the like.

For the preparation of these pharmaceuticals, ordinary additives such as vehicles, disintegrators, binders, lubricants, dyes, and diluents. As the vehicles, lactose, D-mannitol, crystalline cellulose and glucose can be mentioned. Further, there can be mentioned starch and carboxymethylcellulose calcium (CMC-Ca) as the disintegrators, magnesium stearate and talc as the lubricants, and hydroxypropylcellulose (HPC), gelatin and polyvinyl-pirrolidone (PVP) as the binders.

The compound of the invention can be administered to an adult generally in an amount of 0.1 mg to 100 mg a day by parenteral administration and 1 mg to 2,000 mg a day by oral administration. The dosage can be adjusted in consideration of age and conditions of the patient.

The invention is further described by the following non-limiting examples.

### [Example 1] 2-[4-[3-[4-[4-(4-Methoxyphenyl)piperazin-1-ylmethyl]-2-(4-trifluoromethylphenyl)-5-thiazolyl]propionyl]-2-methylphenoxy]propionic acid

### (1) 3-[4-Chloromethyl-2-(4-trifluoromethylphenyl)-5-thiazolyl]-1-(4-hydroxy-3-methylphenyl)propan-1-one

To an ice-cooled THF (10 mL) was added 60% sodium hydride (13 mg, 0.322 mmol). Subsequently, a solution of ethyl 2-[(3-methyl-4-benzyloxy)benzoyl]acetate (92 mg, 0.300 mmol) in THF (3.0 mL) was dropwise added to the mixture for 30 min. The mixture was left to reach room temperature, and then stirred for 30 min. Subsequently, 5-chloromethyl-4-(tetrahydropyran-2-yloxymethyl)-2-(4-trifluromethylphenyl)thiazole (118 mg, 0.301 mmol) was added to the resulting solution, and the mixture was heated under reflux for 20 hours in a nitrogen atmosphere. The mixture was left to reach room temperature and placed under reduced pressure to distill THF off. To the residue was added acetic acid (4.0 mL)/con. hydrochloric acid (1.3 mL), and the mixture was heated under reflux for 20 hours.

The reaction mixture was left to reach room temperature and placed in an ice-cooled water. The mixture was then subjected to extraction with ethyl acetate. The organic portion was taken out, washed subsequently with saturated aqueous sodium hydrogen carbonate solution, water and brine, dried over anhydrous sodium sulfate, and filtered. The filtrate was placed under reduced pressure to distill ethyl acetate off. The residue was purified by silica gel column chromatography (hexane/ethyl acetate=3/1 to 2/1), to yield the titled compound as a pale yellowish white crystalline product (23 mg, yield 18%).

¹H NMR (CDCl₃, 400 MHz) δ: 2.29 (s, 3H), 3.35 (s, 4H), 4.80 (s, 2H), 5.15 (s, 1H), 6.81 (d, 1H, J=8Hz), 7.66 (d, 2H, J=8Hz), 7.75 (dd, 1H, J=2, 8Hz), 7.80 (d, 1H, J=2Hz), 8.00 (d, 2H, J=8Hz).

### (2) 1-(4-Hydroxy-3-methylphenyl)-3-[4-[4-(4-methoxyphenyl)piperazin-1-ylmethyl]-2-(4-trifluoromethylphenyl)-thiazol-5-yl]propan-1-one

In THF (4.0 mL) were dissolved 3-[4-chloromethyl-2-(4-trifluoromethylphenyl)-5-thiazolyl]-1-(4-hydroxy-3-methylphenyl)propan-1-one (22 mg, 0.050 mmol) and 1-(4-methoxyphenyl)piperazine (20 mg, 0.104 mmol). The resulting solution was heated under reflux for 20 hours in a nitrogen atmosphere. The reaction mixture was left to reach room temperature and placed under reduced pressure to distill THF off. The residue was purified by silica gel column chromatography (hexane/ethyl acetate=1/1), to yield the titled compound as colorless oil (26 mg, yield 87%).

¹H NMR (CDCl₃, 400 MHz) δ: 2.25 (s, 3H), 2.7-2.8 (m, 4H), 3.0-3.1 (m, 4H), 3.34 (s, 4H), 3.76 (brs, 3H), 3.79 (s, 2H), 6.94 (d, 1H, J=8Hz), 7.8-7.9 (m, 4H), 7.65 (d, 2H, J=8Hz), 7.72 (d, 1H, J=8Hz), 7.77 (s, 1H), 8.00 (d, 2H, J=8Hz).

### (3) Ethyl 2-[4-[3-[4-[4-(4-methoxyphenyl)piperazin-1-ylmethyl]-2-(4-trifluoromethylphenyl)-5-thiazolyl]propionyl]-2-methylphenoxy]propionate

In acetone (5 mL) were suspended 1-(4-hydroxy-3-methylphenyl)-3-[4-[4-(4-methoxyphenyl)piperazin-1-ylmethyl]-2-(4-trifluoromethylphenyl)thiazol-5-yl]propan-1-one (50 mg, 0.084 mmol), ethyl 2-bromopropionate (23 mg, 0.126 mmol), and potassium carbonate (17 mg, 0.126 mmol). The resulting suspension was stirred for 20 hours at room temperature. The suspension was placed under reduced pressure to distill acetone off. To the residue were added water and ethyl acetate, and the organic portion was taken out. The organic portion was washed successively with water and brine, dried over anhydrous sodium sulfate, and filtered. The filtrate was placed under reduced pressure to distill ethyl acetate off. The residue was purified by silica gel column chromatography (hexane/ethyl acetate=2/1), to yield the titled compound as colorless oil (53 mg, yield 91%).

¹H NMR (CDCl₃, 400 MHz) δ: 1.23 (t, 3H, J=7Hz), 1.65 (d, 3H, J=6Hz), 2.29 (s, 3H), 2.7-2.8 (m, 4H), 3.0-3.1 (m, 4H), 3.3-3.4 (m, 4H), 3.76 (s, 3H), 3.78 (s, 2H), 4.19 (q, 2H, J=7Hz), 4.80 (q, 1H, J=6Hz), 6.66 (d, 1H, J=8Hz), 6.8-6.9 (m, 4H), 7.65 (d, 2H, J=8Hz), 7.75 (dd, 1H, J=2, 8Hz), 7.80 (d, 1H, J=2Hz), 8.00 (d, 2H, J=8Hz).

### (4) 2-[4-[3-[4-[4-(4-Methoxyphenyl)piperazin-1-ylmethyl]-2-(4-trifluoromethylphenyl)-5-thiazolyl]propionyl]-2-methylphenoxy]propionic acid

The above-obtained ester (52 mg, 0.075 mmol) was suspended in an ethanol/water mixture (3 mL/1 mL). Lithium hydroxide monohydrate (6 mg, 0.150 mmol) was added to the resulting suspension. The mixture was heated under reflux for 2 hours. After disappearance of the starting compound was confirmed, the reaction mixture was left to reach room temperature. Then, the mixture was adjusted to pH 5-6 by addition of ice and 1N HCl. After addition of ethyl acetate, the organic portion was taken out. The organic portion was washed successively with water and brine, dried over anhydrous sodium sulfate, and filtered. The filtrate was placed under reduced pressure to distill ethyl acetate off, to give the titled compound as colorless amorphous residue (40 mg, yield 80%).

¹H NMR (CD₃OD, 400 MHz) δ: 1.59 (d, 3H, J=6Hz), 2.27 (s, 3H), 3.2-3.4 (m, 12H), 3.74 (s, 3H), 4.35 (s, 2H), 4.66 (q, 1H, J=6Hz), 6.7-7.0 (m, 5H), 7.7-7.9 (m, 4H), 8.10 (d, 2H, J=8Hz).

### [Example 2] 4-[3-[4-[4-(4-Methoxyphenyl)piperazin-1-ylmethyl]-2-(4-trifluoromethylphenyl)-5-thiazolyl]propionyl]-2-methylphenoxyacetic acid

### (1) Ethyl 4-[3-[4-[4-(4-methoxyphenyl)piperazin-1-ylmethyl]-2-(4-trifluoromethylphenyl)-5-thiazolyl]propionyl]-2-methylphenoxyacetate

In acetone (3 mL) were suspended 1-(4-hydroxy-3-methylphenyl)-3-[4-[4-(4-methoxyphenyl)piperazin-1-ylmethyl]-2-(4-trifluoromethylphenyl)thiazol-5-yl]propan-1-one prepared in Example 1-(2) (25 mg, 0.042 mmol), ethyl 2-bromoacetate (12 mg, 0.071 mmol), and potassium carbonate (12 mg, 0.088 mmol). The resulting suspension was stirred for 20 hours at room temperature, and placed under reduced under reduced pressure to distill acetone off. To the residue were added water and ethyl acetate, and the organic portion was taken out. The organic portion was washed successively with saturated aqueous sodium hydrogen carbonate solution, water and brine, dried over anhydrous sodium sulfate, and filtered. The filtrate was placed under reduced pressure to distill ethyl acetate off. The residue was purified by silica gel column chromatography (hexane/ethyl acetate=3/1), to yield the titled compound as colorless oil (28 mg, yield 97%).

¹H NMR (CDCl₃, 400 MHz) δ: 1.29 (t, 3H, J=7Hz), 2.30 (s, 3H), 2.7-2.8 (m, 4H), 3.0-3.1 (m, 4H), 3.3-3.4 (m, 4H), 3.76 (s, 3H), 3.79 (s, 2H), 4.26 (q, 2H, J=7Hz), 4.68 (s, 2H), 6.69 (d, 1H, J=8Hz), 6.8-6.9 (m, 4H), 7.65 (d, 2H, J=8Hz), 7.7-7.9 (m, 2H), 8.00 (d, 2H, J=8Hz).

### (2) 4-[3-[4-[4-(4-Methoxyphenyl)piperazin-1-yl-methyl]-2-(4-trifluoromethylphenyl)-5-thiazolyl]propionyl]-2-methylphenoxyacetic acid

The titled compound was prepared as a white crystalline product by procedures similar to the procedures of Example 1-(4). Yield 79%.

¹H NMR (CD₃OD, 400 MHz) δ: 2.28 (s, 3H), 3.2-3.6 (m, 12H), 3.74 (s, 3H), 4.37 (s, 2H), 4.58 (s, 2H), 6.8-7.0 (m, 5H), 7.7-7.9 (m, 4H), 8.11 (d, 2H, J=8Hz).

### [Example 3] 2-[4-[3-[4-[4-(4-Methoxyphenyl)piperazin-1-ylmethyl]-2-(4-trifluoromethylphenyl)-5-thiazolyl]propionyl]-2-methylphenoxy]-2-methylpropionic acid

### (1) Ethyl 2-[4-[3-[4-[4-(4-methoxyphenyl)piperazin-1-ylmethyl]-2-(4-trifluoromethylphenyl)-5-thiazolyl]propionyl]-2-methylphenoxy]-2-methylpropionate

In 2-butanone (4 mL) were suspended 1-(4-hydroxy-3-methylphenyl)-3-[4-[4-(4-methoxyphenyl)piperazin-1-ylmethyl]-2-(4-trifluoromethylphenyl)thiazol-5-yl]propan-1-one (47 mg, 0.079 mmol), ethyl 2-bromo-2-methylpropionate (46 mg, 0.236 mmol), and potassium carbonate (33 mg, 0.236 mmol). The resulting suspension was heated under reflux for 20 hours. The suspension was then placed under reduced pressure to distill 2-butanone off. To the residue were added water and ethyl acetate, and the organic portion was taken out. The organic portion was washed successively with water and brine, dried over anhydrous sodium sulfate, and filtered. The filtrate was placed under reduced pressure to distill ethyl acetate off. The reside was purified by silica gel column chromatography (hexane/ethyl acetate=3/1 to 2/1), to yield the titled compound as a white crystalline product (28 mg, yield 95%).

¹H NMR (CDCl₃, 400 MHz) δ: 1.20 (t, 3H, J=7Hz), 1.64 (s, 6H), 2.24 (s, 3H), 2.7-2.8 (m, 4H), 3.0-3.1 (m, 4H), 3.3-3.4 (m, 4H), 3.76 (s, 3H), 3.78 (s, 2H), 4.20 (q, 2H, J=7Hz), 6.60 (d, 1H, J=8Hz), 6.8-6.9 (m, 4H), 7.65 (d, 2H, J=8Hz), 7.71 (dd, 1H, J=2, 8Hz), 7.79 (d, 1H, J=2Hz), 8.00 (d, 2H, J=8Hz).

### (2) 2-[4-[3-[4-[4-(4-Methoxyphenyl)piperazin-1-yl-methyl]-2-(4-trifluoromethylphenyl)-5-thiazolyl]propionyl]-2-methylphenoxy]-2-methylpropionic acid

The titled compound was prepared as a white crystalline product by procedures similar to the procedures of Example 1-(4). Yield 71%.

¹H NMR (CD₃OD, 400 MHz) δ: 1.61 (s, 6H), 2.23 (s, 3H), 3.2-3.6 (m, 12H), 3.74 (s, 3H), 4.35 (s, 2H), 6.8-7.0 (m, 5H), 7.7-7.9 (m, 4H), 8.11 (d, 2H, J=8Hz).

### [Example 4] 2-[4-[3-[4-[4-(3-Methoxyphenyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(3-methoxyphenyl)piperazin-1-ylmethyl]-2-(4-trifluoromethylphenyl)-thiazol-5-yl]propan-1-one

The titled compound was prepared by procedures similar to the procedures of Example 1-(2) using 1-(3-methoxyphenyl)piperazine. Yield 81%.

¹H NMR (CDCl₃, 400 MHz) δ: 2.25 (s, 3H), 2.7-2.8 (m, 4H), 3.1-3.2 (m, 4H), 3.34 (s, 4H), 3.78 (s, 2H), 3.78 (s, 3H), 6.3-6.4 (m, 2H), 6.52 (1H, dd, J=2, 8Hz), 6.74 (d, 1H, J=8Hz), 7.15 (t, 1H, J=8Hz), 7.65 (d, 2H, J=8Hz), 7.71 (dd, 1H, J=2, 8Hz), 7.77 (d, 1H, J=2Hz), 8.00 (d, 2H, J=8Hz).

### (2) Ethyl 2-[4-[3-[4-[4-(3-methoxyphenyl)piperazin-1-ylmethyl]-2-(4-trifluoromethylphenyl)-5-thiazolyl]propionyl]-2-methylphenoxy]-2-methylpropionate

The titled compound was prepared by procedures similar to the procedures of Example 3-(1). Yield 92%.

¹H NMR (CDCl₃, 400 MHz) δ: 1.20 (t, 3H, J=7Hz), 1.64 (s, 6H), 2.24 (s, 3H), 2.7-2.8 (m, 4H), 3.1-3.3 (m, 4H), 3.35 (s, 4H), 3.78 (s, 2H), 3.78 (s, 3H), 4.20 (q, 2H, J=7Hz), 6.3-6.5 (m, 2H), 6.52 (dd, 1H, J=2, 8Hz), 6.60 (d, 1H, J=8Hz), 7.15 (t, 1H, J=8Hz), 7.65 (d, 2H, J=8Hz), 7.70 (dd, 1H, J=2, 8Hz), 7.79 (d, 1H, J=2Hz), 8.00 (d, 2H, J=8Hz).

### (3) 2-[4-[3-[4-[4-(3-Methoxyphenyl)piperazin-1-ylmethyl]-2-(4-trifluoromethylphenyl)-5-thiazolyl]propionyl]-2-methylphenoxy]-2-methylpropionic acid

The titled compound was prepared as a pale yellow amorphous product by procedures similar to the procedures of Example 1-(4). Yield 95%.

¹H NMR (CD₃OD, 400 MHz) δ: 1.60 (s, 6H), 2.22 (s, 3H), 3.1-3.5 (m, 12H), 3.75 (s, 3H), 4.24 (s, 2H), 6.4-6.6 (m, 3H), 6.82 (d, 1H, J=8Hz), 7.15 (t, 1H, J=8Hz), 7.7-7.8 (m, 4H), 8.11 (d, 2H, J=8Hz).

### [Example 5] 2-[4-[3-[4-[4-(4-Trifluoromethylphenyl)-piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-trifluoromethylphenyl)piperazin-1-ylmethyl]-2-(4-trifluoromethylphenyl)thiazol-5-yl]propan-1-one

The titled compound was prepared as colorless oil by procedures similar to the procedures of Example 1-(2) using 1-(4-trifluoromethylphenyl)piperazine. Yield 90%.

¹H NMR (CDCl₃, 400 MHz) δ: 2.25 (s, 3H), 2.7-2.8 (m, 4H), 3.2-3.3 (m, 4H), 3.36 (brs, 4H), 3.78 (s, 2H), 5.2 (brs. 1H), 6.77 (d, 1H, J=8Hz), 6.89 (d, 2H, J=8Hz), 7.46 (d, 2H, J=8Hz), 7.65 (d, 2H, J=8Hz), 7.74 (dd, 1H, J=2, 8Hz), 7.79 (d, 1H, J=2Hz), 8.00 (d, 2H, J=8Hz).

### (2) Ethyl 2-[4-[3-[4-[4-(4-trifluoromethylphenyl)-piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 85%.

¹H NMR (CDCl₃, 400 MHz) δ: 1.20 (t, 3H, J=7Hz), 1.63 (s, 6H), 2.23 (s, 3H), 2.6-2.8 (m, 4H), 3.2-3.3 (m, 4H), 3.35 (brs, 4H), 3.78 (s, 2H), 4.20 (q, 2H, J=7Hz), 6.60 (d, 1H, J=8Hz), 6.89 (d, 2H, J=8Hz), 7.46 (d, 2H, J=8Hz), 7.65 (d, 2H, J=8Hz), 7.70 (dd, 1H, J=2, 8Hz), 7.78 (d, 1H, J=2Hz), 8.00 (d, 2H, J=8Hz).

### (3) 2-[4-[3-[4-[4-(4-Trifluoromethylphenyl)piperazin-1-ylmethyl]-2-(4-trifluoromethylphenyl)-5-thiazolyl]propionyl]-2-methylphenoxy]-2-methylpropionic acid

The titled compound was prepared as a pale yellow amorphous product by procedures similar to the procedures of Example 1-(4). Yield 79%.

¹H NMR (CD₃OD, 400 MHz) δ: 1.60 (s, 6H), 2.21 (s, 3H), 3.0-3.5 (m, 12H), 4.11 (s, 2H), 4.80 (d, 1H, J=8Hz), 7.06 (d, 2H, J=8Hz), 7.49 (d, 2H, J=8Hz), 7.7-7.9 (m, 4H), 8.10 (d, 2H, J=8Hz).

### [Example 6] 2-[4-[3-[4-[4-(4-Methylphenyl)piperazin-1-yl-methyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-methylphenyl)piperazin-1-ylmethyl]-2-(4-trifluoromethylphenyl)-thiazol-5-yl]propan-1-one

The titled compound was prepared as colorless oil by procedures similar to the procedures of Example 1-(2) using 1-(4-methylphenyl)piperazine. Yield 91%.

¹H NMR (CDCl₃, 400 MHz) δ: 2.25 (s, 3H), 2.26 (s, 3H), 2.7-2.8 (m, 4H), 3.2-3.3 (m, 4H), 3.35 (brs, 4H), 3.78 (s, 2H), 6.73 (d, 1H, J=8Hz), 6.81 (d, 2H, J=8Hz), 7.05 (d, 2H, J=8Hz), 7.65 (d, 2H, J=8Hz), 7.70 (dd, 1H, J=2, 8Hz), 7.76 (d, 1H, J=2Hz), 8.00 (d, 2H, J=8Hz).

### (2) Ethyl 2-[4-[3-[4-[4-(4-methylphenyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 1-(3). Yield 77%.

¹H NMR (CDCl₃, 400 MHz) δ: 1.20 (t, 3H, J=7Hz), 1.64 (s, 6H), 2.24 (s, 3H), 2.26 (s, 3H), 2.7-2.8 (m, 4H), 3.1-3.2 (m, 4H), 3.35 (brs, 4H), 3.78 (s, 2H), 4.20 (q, 2H, J=7Hz), 6.60 (d, 1H, J=8Hz), 6.82 (d, 2H, J=8Hz), 7.06 (d, 2H, J=8Hz), 7.65 (d, 2H, J=8Hz), 7.70 (dd, 1H, J=2, 8Hz), 7.79 (d, 1H, J=2Hz), 8.00 (d, 2H, J=8Hz).

### (3) 2-[4-[3-[4-[4-(4-Methylphenyl)piperazin-1-ylmethyl]-2-(4-trifluoromethylphenyl)-5-thiazolyl]propionyl]-2-methylphenoxy]-2-methylpropionic acid

The titled compound was prepared as a brown amorphous product by procedures similar to the procedures of Example 1-(4). Yield 97%.

¹H NMR (CD₃OD, 400 MHz) δ: 1.60 (s, 6H), 2.21 (s, 3H), 2.24 (s, 3H), 3.2-3.4 (m, 12H), 4.25 (s, 2H), 6.81 (d, 1H, J=8Hz), 6.89 (d, 2H, J=8Hz), 7.07 (d, 2H, J=8Hz), 7.7-7.8 (m, 4H), 8.10 (d, 2H, J=8Hz).

### [Example 7] 2-[4-[3-[4-[4-(4-Methoxybenzyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-methoxybenzyl)piperazin-1-ylmethyl]-2-(4-trifluoromethylphenyl)-thiazol-5-yl]propan-1-one

The titled compound was prepared as colorless oil by procedures similar to the procedures of Example 1-(2) using 1-(4-methoxybenzyl)piperazine. Yield 72%.

¹H NMR (CDCl₃, 400 MHz) δ: 2.26 (s, 3H), 2.3-2.8 (m, 8H), 3.27 (brs, 4H), 3.43 (s, 2H), 3.72 (s, 2H), 3.78 (s, 3H), 6.6-6.7 (m, 1H), 6.83 (d, 2H, J=8Hz), 7.19 (d, 2H, J=8Hz), 7.6-7.7 (m, 3H), 7.75 (s, 1H), 7.97 (d, 2H, J=8Hz).

### (2) Ethyl 2-[4-[3-[4-[4-(4-methoxybenzyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 84%.

¹H NMR (CDCl₃, 400 MHz) δ: 1.21 (t, 3H, J=7Hz), 1.66 (s, 6H), 2.27 (s, 3H), 2.3-2.8 (m, 8H), 3,3-3.4 (m, 4H), 3.43 (s, 2H), 3.72 (s, 2H), 3.79 (s, 3H), 4.22 (q, 2H, J=7Hz), 6.62 (d, 1H, J=8Hz), 6.83 (d, 2H, J=8Hz), 7.20 (d, 2H, J=8Hz), 7.64 (d, 2H, J=8Hz), 7.72 (dd, 1H, J=2, 8Hz), 7.80 (d, 1H, J=2Hz), 7.98 (d, 2H, J=8Hz).

### (3) 2-[4-[3-[4-[4-(4-Methoxybenzyl)piperazin-1-ylmethyl]-2-(4-trifluoromethylphenyl)-5-thiazolyl]propionyl]-2-methylphenoxy]-2-methylpropionic acid

The titled compound was prepared as a brown amorphous product by procedures similar to the procedures of Example 1-(4). Yield 65%.

¹H NMR (CD₃OD, 400 MHz) δ: 1.64 (s, 6H), 2.25 (s, 3H), 2.5-3.4 (m, 12H), 3.70 (s, 2H), 4.06 (s, 2H), 6.87 (d, 1H, J=8Hz), 6.95 (d, 2H, J=8Hz), 7.38 (d, 2H, J=8Hz), 7.7-7.8 (m, 4H), 8.07 (d, 2H, J=8Hz).

### [Example 8] 2-[4-[3-[4-[4-(4-Methoxybenzoyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-methoxybenzoyl)piperazin-1-ylmethyl]-2-(4-trifluoromethylphenyl)thiazol-5-yl]propan-1-one

The titled compound was prepared as colorless oil by procedures similar to the procedures of Example 1-(2) using 1-(4-methoxybenzoyl)piperazine. Yield: quantitative.

¹H NMR (CDCl₃, 400 MHz) δ: 2.27 (s, 3H), 2.5-2.7 (m, 4H), 3.32 (brs, 4H), 3.4-3.8 (m, 4H), 3.75 (s, 2H), 3.82 (s, 3H), 6.78 (d, 1H, J=8Hz), 6.8-7.0 (m, 2H), 7.3-7.4 (m, 2H), 7.64 (d, 2H, J=8Hz), 7.69 (d, 1H, J=8Hz), 7.78 (s, 1H), 7.98 (d, 2H, J=8Hz).

### (2) Ethyl 2-[4-[3-[4-[4-(4-methoxybenzoyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 91%.

¹H NMR (CDCl₃, 400 MHz) δ: 1.21 (t, 3H, J=7Hz), 1.66 (s, 6H), 2.27 (s, 3H), 2.4-2.7 (m, 4H), 3.32 (brs, 4H), 3.4-3.8 (m, 4H), 3.75 (s, 2H), 3.82 (s, 3H), 4.24 (q, 2H, J=7Hz), 6.61 (d, 1H, J=8Hz), 6.8-7.0 (m, 2H), 7.3-7.4 (m, 2H), 7.65 (d, 2H, J=8Hz), 7.69 (dd, 1H, J=2, 8Hz), 7.78 (d, 1H, J=2Hz), 7.98 (d, 2H, J=8Hz).

### (3) 2-[4-[3-[4-[4-(4-Methoxybenzoyl)piperazin-1-ylmethyl]-2-(4-trifluoromethylphenyl)-5-thiazolyl]propionyl]-2-methylphenoxy]-2-methylpropionic acid

The titled compound was prepared as a colorless amorphous product by procedures similar to the procedures of Example 1-(4). Yield 66%.

¹H NMR (CD₃OD, 400 MHz) δ: 1.63 (s, 6H), 2.24 (s, 3H), 2.6-3.8 (m, 12H), 3.83 (s, 3H), 3.85 (s, 2H), 6.80 (d, 1H, J=8Hz), 6.98 (d, 2H, J=8Hz), 7.38 (d, 2H, J=8Hz), 7.7-7.8 (m, 4H), 8.07 (d, 2H, J=8Hz).

### [Example 9] 2-[4-[3-[4-[4-(2-Methoxyphenyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(2-methoxyphenyl)piperazin-1-ylmethyl]-2-(4-trifluoromethylphenyl)-thiazol-5-yl]propan-1-one

The titled compound was prepared as colorless oil by procedures similar to the procedures of Example 1-(2) using 1-(2-methoxyphenyl)piperazine. Yield 89%.

¹H NMR (CDCl₃, 400 MHz) δ: 2.26 (s, 3H), 2.7-2.8 (m, 4H), 3.0-3.1 (m, 4H), 3.35 (brs, 4H), 3.80 (s, 2H), 3.85 (s, 3H), 6.76 (d, 1H, J=8Hz), 6.8-7.0 (m, 4H), 7.65 (d, 2H, J=8Hz), 7.73 (d, 1H, J=2, 8Hz), 7.78 (d, 1H, J=2Hz), 8.00 (d, 2H, J=8Hz).

### (2) Ethyl 2-[4-[3-[4-(4-(2-methoxyphenyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 91%.

¹H NMR (CDCl₃, 400 MHz) δ: 1.20 (t, 3H, J=7Hz), 1.64 (s, 6H), 2.25 (s, 3H), 2.7-2.8 (m, 4H), 3.0-3.1 (m, 4H), 3.36 (brs, 4H), 3.80 (s, 2H), 3.85 (s, 3H), 4.20 (q, 2H, J=7Hz), 6.61 (d, 1H, J=8Hz), 6.85 (d, 1H, J=8Hz), 6.9-7.0 (m, 3H), 7.65 (d, 2H, J=8Hz), 7.72 (d, 1H, J=2, 8Hz), 7.80 (d, 1H, J=2Hz), 8.00 (d, 2H, J=8Hz).

### (3) 2-[4-[3-[4-[4-(2-Methoxyphenyl)piperazin-1-ylmethyl]-2-(4-trifluoromethylphenyl)-5-thiazolyl]propionyl]-2-methylphenoxy]-2-methylpropionic acid

The titled compound was prepared as a colorless amorphous product by procedures similar to the procedures of Example 1-(4). Yield 78%.

¹H NMR (CD₃OD, 400 MHz) δ: 1.59 (s, 6H), 2.23 (s, 3H), 3.2-3.5 (m, 12H), 3.86 (s, 3H), 4.33 (s, 2H), 6.83 (d, 1H, J=8Hz), 6.9-7.1 (m, 4H), 7.7-7.8 (m, 4H), 8.12 (d, 2H, J=8Hz).

### [Example 10] 2-[4-[3-[4-[4-(4-Fluorophenyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-fluorophenyl)piperazin-1-ylmethyl]-2-(4-trifluoromethylphenyl)-thiazol-5-yl]propan-1-one

The titled compound was prepared as colorless oil by procedures similar to the procedures of Example 1-(2) using 1-(4-fluorophenyl)piperazine. Yield 83%.

¹H NMR (CDCl₃, 400 MHz) δ: 2.25 (s, 3H), 2.7-2.8 (m, 4H), 3.0-3.2 (m, 4H), 3.35 (s, 4H), 3.79 (s, 2H), 6.75 (d, 1H, J=8Hz), 6.8-7.0 (m, 4H), 7.65 (d, 2H, J=8Hz), 7.72 (dd, 1H, J=2, 8Hz), 7.79 (d, 1H, J=2Hz), 8.00 (d, 2H, J=8Hz).

### (2) Ethyl 2-[4-[3-[4-[4-(4-fluorophenyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 91%.

¹H NMR (CDCl₃, 400 MHz) δ: 1.20 (t, 3H, J=7Hz), 1.64 (s, 6H), 2.24 (s, 3H), 2.7-2.8 (m, 4H), 3.0-3.1 (m, 4H), 3.35 (brs, 4H), 3.78 (s, 2H), 4.20 (q, 2H, J=7Hz), 6.60 (d, 1H, J=8Hz), 6.8-7.0 (m, 4H), 7.65 (d, 2H, J=8Hz), 7.70 (dd, 1H, J=2, 8Hz), 7.79 (d, 1H, J=2Hz), 8.00 (d, 2H, J=8Hz).

### (3) 2-[4-[3-[4-[4-(4-Fluorophenyl)piperazin-1-ylmethyl]-2-(4-trifluoromethylphenyl)-5-thiazolyl]propionyl]-2-methylphenoxy]-2-methylpropionic acid

The titled compound was prepared as a colorless amorphous product by procedures similar to the procedures of Example 1-(4). Yield 82%.

¹H NMR (CD₃OD, 400 MHz) δ: 1.60 (s, 6H), 2.22 (s, 3H), 3.1-3.5 (m, 12H), 4.16 (s, 2H), 6.82 (d, 1H, J=8Hz), 6.9-7.0 (m, 4H), 7.7-7.8 (m, 4H), 8.10 (d, 2H, J=8Hz).

### [Example 11] 2-[4-[3-[4-[4-(4-Isopropylphenyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-isopropylphenyl)piperazin-1-ylmethyl]-2-(4-trifluoromethylphenyl)thiazol-5-yl]propan-1-one

The titled compound was prepared as colorless oil by procedures similar to the procedures of Example 1-(2) using 1-(4-isopropylphenyl)piperazine. Yield: quantitative.

¹H NMR (CDCl₃, 400 MHz) δ: 1.21 (d, 6H, J=6Hz), 2.25 (s, 3H), 2.7-2.8 (m, 4H), 2.83 (dq, 1H, J=6Hz, J=6Hz), 3.1-3.2 (m, 4H), 3.35 (brs, 4H), 3.78 (s, 2H), 6.76 (d, 1H, J=8Hz), 6.84 (d, 2H, J=8Hz), 7.05 (d, 2H, J=8Hz), 7.64 (d, 2H, J=8Hz), 7.73 (dd, 1H, J=1, 8Hz), 7.78 (d, 1H, J=1Hz), 8.00 (d, 2H, J=8Hz).

### (2) Ethyl 2-[4-[3-[4-[4-(4-isopropylphenyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 61%.

¹H NMR (CDCl₃, 400 MHz) δ: 1.20 (t, 3H, J=7Hz), 1.21 (d, 6H, J=6Hz), 1.64 (s, 6H), 2.24 (s, 3H), 2.7-2.8 (m, 4H), 2.83 (dq, 1H, J=6Hz, J=6Hz), 3.1-3.2 (m, 4H), 3.35 (brs, 4H), 3.78 (s, 2H), 4.20 (q, 2H, J=7Hz), 6.60 (d, 1H, J=8Hz), 6.84 (d, 2H, J=8Hz), 7.11 (d, 2H, J=8Hz), 7.65 (d, 2H, J=8Hz), 7.70 (dd, 1H, J=2, 8Hz), 7.79 (d, 1H, J=2Hz), 8.00 (d, 2H, J=8Hz).

### (3) 2-[4-[3-[4-[4-(4-Isopropylphenyl)piperazin-1-ylmethyl]-2-(4-trifluoromethylphenyl)-5-thiazolyl]propionyl]-2-methylphenoxy]-2-methylpropionic acid

The titled compound was prepared as a pale brown amorphous product by procedures similar to the procedures of Example 1-(4). Yield 84%.

¹H NMR (CD₃OD, 400 MHz) δ: 1.20 (d, 6H, J=6Hz), 1.60 (s, 6H), 2.23 (s, 3H), 2.82 (dq, 1H, J=6Hz, J=6Hz), 3.2-3.5 (m, 12H), 4.25 (s, 2H), 6.82 (d, 1H, J=8Hz), 6.93 (d, 2H, J=8Hz), 7.13 (d, 2H, J=8Hz), 7.7-7.8 (m, 4H), 8.10 (d, 2H, J=8Hz).

### [Example 12] 2-[4-[3-[4-[4-(3,4-Dimethoxyphenyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(3,4-dimethoxyphenyl)piperazin-1-ylmethyl]-2-(4-trifluoromethylphenyl)thiazol-5-yl]propan-1-one

The titled compound was prepared as colorless oil by procedures similar to the procedures of Example 1-(2) using 1-(3,4-dimethoxyphenyl)piperazine. Yield 84%.

¹H NMR (CDCl₃, 400 MHz) δ: 2.26 (s, 3H), 2.7-2.8 (m, 4H), 3.0-3.1 (m, 4H), 3.35 (brs, 4H), 3.79 (s, 2H), 3.83 (s, 3H), 3.85 (s, 3H), 6.42 (dd, 1H, J=2, 8Hz), 6.55 (d, 1H, J=8Hz), 6.76 (d, 1H, J=8Hz), 6.77 (d, 1H, J=8Hz), 7.65 (d, 2H, J=8Hz), 7.74 (dd, 1H, J=2, 8Hz), 7.79 (d, 1H, J=2Hz), 8.00 (d, 2H, J=8Hz).

### (2) Ethyl 2-[4-[3-[4-[4-(3,4-dimethoxyphenyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 92%.

¹H NMR (CDCl₃, 400 MHz) δ: 1.20 (t, 3H, J=7Hz), 1.64 (s, 6H), 2.24 (s, 3H), 2.7-2.8 (m, 4H), 3.0-3.1 (m, 4H), 3.35 (brs, 4H), 3.79 (s, 2H), 3.83 (s, 3H), 3.85 (s, 3H), 4.20 (q, 2H, J=7Hz), 6.42 (dd, 1H, J=2, 8Hz), 6.56 (d, 1H, J=2Hz), 6.61 (d, 1H, J=8Hz), 6.78 (d, 1H, J=8Hz), 7.65 (d, 2H, J=8Hz), 7.71 (dd, 1H, J=2, 8Hz), 7.79 (d, 1H, J=2Hz), 8.00 (d, 2H, J=8Hz).

### (3) 2-[4-[3-[4-[4-(3,4-Dimethoxyphenyl)piperazin-1-ylmethyl]-2-(4-trifluoromethylphenyl)-5-thiazolyl]propionyl]-2-methylphenoxy]-2-methylpropionic acid

The titled compound was prepared as a pale brown amorphous product by procedures similar to the procedures of Example 1-(4). Yield 88%.

¹H NMR (CD₃OD, 400 MHz) δ: 1.60 (s, 6H), 2.23 (s, 3H), 3.2-3.5 (m, 12H), 3.77 (s, 3H), 3.81 (s, 3H), 4.25 (s, 2H), 6.52 (dd, 1H, J=2, 8Hz), 6.68 (d, 1H, J=2Hz), 6.82 (d, 1H, J=8Hz), 6.86 (d, 1H, J=8Hz), 7.7-7.8 (m, 4H), 8.10 (d, 2H, J=8Hz).

### [Example 13] 2-[4-[3-[4-[4-(4-Ethoxyphenyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-ethoxyphenyl)piperazin-1-ylmethyl]-2-(4-trifluoromethylphenyl)-thiazol-5-yl]propan-1-one

The titled compound was prepared as colorless oil by procedures similar to the procedures of Example 1-(2) using 1-(4-ethoxyphenyl)piperazine. Yield 84%.

¹H NMR (CDCl₃, 400 MHz) δ: 1.38 (t, 3H, J=7Hz), 2.25 (s, 3H), 2.7-2.8 (m, 4H), 3.0-3.1 (m, 4H), 3.35 (brs, 4H), 3.78 (s, 2H), 3.98 (q, 2H, J=7Hz), 6.76 (d, 1H, J=8Hz), 6.8-6.9 (m, 4H), 7.65 (d, 2H, J=8Hz), 7.73 (dd, 1H, J=2, 8Hz), 7.78 (d, 1H, J=2Hz), 8.00 (d, 2H, J=8Hz).

### (2) Ethyl 2-[4-[3-[4-[4-(4-ethoxyphenyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 1-(3). Yield 78%.

¹H NMR (CDCl₃, 400 MHz) δ: 1.20 (t, 3H, J=7Hz), 1.38 (t, 3H, J=7Hz), 1.64 (s, 6H), 2.24 (s, 3H), 2.7-2.8 (m, 4H), 3.0-3.1 (m, 4H), 3.35 (brs, 4H), 3.78 (s, 2H), 3.98 (q, 2H, J=7Hz), 4.20 (q, 2H, J=7Hz), 6.60 (d, 1H, J=8Hz), 6.8-6.9 (m, 4H), 7.65 (d, 2H, J=8Hz), 7.71 (dd, 1H, J=2, 8Hz), 7.79 (d, 1H, J=2Hz), 8.00 (d, 2H, J=8Hz).

### (3) 2-[4-[3-[4-[4-(4-Ethoxyphenyl)piperazin-1-ylmethyl]-2-(4-trifluoromethylphenyl)-5-thiazolyl]propionyl]-2-methylphenoxy]-2-methylpropionic acid

The titled compound was prepared as a pale brown amorphous product by procedures similar to the procedures of Example 1-(4). Yield 89%.

¹H NMR (CD₃OD, 400 MHz) δ: 1.25 (t, 3H, J=7Hz), 1.60 (s, 6H), 2.22 (s, 3H), 3.2-3.4 (m, 12H), 3.35 (s, 4H), 3.97 (q, 2H, J=7Hz), 4.26 (s, 2H), 6.8-7.0 (m, 5H), 7.7-7.8 (m, 4H), 8.09 (d, 2H, J=8Hz).

### [Example 14] 2-[4-[3-[4-[4-(4-Methoxyphenyl)piperazin-1-ylmethyl]-2-(4-trifluoromethylphenyl)-5-thiazolyl]propionyl]phenoxy]-2-methylpropionic acid

### (1) 1-(4-Hydroxyphenyl)-3-[4-[4-(4-methoxyphenyl)-piperazin-1-ylmethyl]-2-(4-trifluoromethylphenyl)thiazol-5-yl]propan-1-one

To an ice-cooled THF (10 mL) was added 60% sodium hydride (10 mg, 0.254 mmol). Subsequently, a solution of ethyl 2-[(4-benzyloxy)benzoyl] acetate (69 mg, 0.231 mmol) in THF (3.0 mL) was dropwise added to the mixture for 30 min. The mixture was left to reach room temperature, and then stirred for 30 min. Subsequently, 5-chloromethyl-4-(tetrahydropyran-2-yloxymethyl)-2-(4-trifluromethylphenyl)thiazole (91 mg, 0.231 mmol) was added to the resulting solution, and the mixture was heated under reflux for 20 hours in a nitrogen atmosphere. The mixture was left to reach room temperature and placed under reduced pressure to distill THF off. To the residue was added acetic acid (2 mL)/con. hydrochloric acid (2 mL), and the mixture was heated under reflux for 20 hours.

The reaction mixture was placed in an ice-cooled water. The mixture was then subjected to extraction with ethyl acetate. The organic portion was taken out, washed subsequently with saturated aqueous sodium hydrogen carbonate solution, water and brine, dried over anhydrous sodium sulfate, and filtered. The filtrate was placed under reduced pressure to distill ethyl acetate off. The residue was purified by silica gel column chromatography (hexane/ethyl acetate=3/1 to 1/3), to yield the titled compound as a pale yellowish white crystalline product (18 mg, yield 18%).

¹H NMR (CDCl₃, 400 MHz) δ: 3.3-3.4 (m, 4H), 4.80 (s, 2H), 5.44 (brs, 1H), 6.88 (d, 2H, J=8Hz), 7.66 (d, 2H, J=8Hz), 7.92 (d, 2H, J=8Hz), 8.00 (d, 2H, J=8Hz).

### (2) 1-(4-Hydroxyphenyl)-3-[4-[4-(4-methoxyphenyl)-piperazin-1-ylmethyl]-2-(4-trifluoromethylphenyl)thiazol-5-yl]propan-1-one

The titled compound was prepared as colorless oil by procedures similar to the procedures of Example 1-(2) using 1-(4-methoxyphenyl)piperazine. Yield 96%.

¹H NMR (CDCl₃, 400 MHz) δ: 2.7-2.8 (m, 4H), 3.0-3.1 (m, 4H), 3.3-3.4 (m, 4H), 3.76 (s, 3H), 3.79 (s, 2H), 6.7-6.9 (m, 6H), 7.64 (d, 2H, J=8Hz), 7.85 (d, 2H, J=8Hz), 7.78 (d, 2H, J=8Hz).

### (3) Ethyl 2-[4-[3-[4-[4-(4-methoxyphenyl)piperazin-1-ylmethyl]-2-(4-trifluoromethylphenyl)-5-thiazolyl]propionyl]phenoxy]-2-methylpropionate

The titled compound was prepared as colorless oil by procedures similar to the procedures of Example 3-(1). Yield 78%.

¹H NMR (CDCl₃, 400 MHz) δ: 1.20 (t, 3H, J=7Hz), 1.64 (s, 6H), 2.6-2.8 (m, 4H), 3.0-3.1 (m, 4H), 3.35 (brs, 4H), 3.76 (s, 3H), 3.78 (s, 2H), 4.20 (q, 2H, J=7Hz), 6.8-6.9 (m, 6H), 7.65 (d, 2H, J=8Hz), 7.8-7.9 (m, 2H), 7.78 (d, 2H, J=8Hz).

### (4) 2-[4-[3-[4-[4-(4-Methoxyphenyl)piperazin-1-ylmethyl]-2-(4-trifluoromethylphenyl)-5-thiazolyl]propionyl]phenoxy]-2-methylpropionic acid

The titled compound was prepared as a pale yellow amorphous product by procedures similar to the procedures of Example 1-(4). Yield 89%.

¹H NMR (CD₃OD, 400 MHz) δ: 1.59 (s, 6H), 3.2-3.5 (m, 12H), 3.74 (s, 3H), 4.28 (s, 2H), 6.8-7.0 (m, 6H), 7.76 (d, 2H, J=8Hz), 7.8-8.0 (m, 2H), 8.10 (d, 2H, J=8Hz).

### [Example 15] 2-[4-[3-[4-[4-(4-Trifluoromethoxyphenyl)-piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-trifluoromethoxyphenyl)piperazin-1-ylmethyl]-2-(4-trifluoromethylphenyl)thiazol-5-yl]propan-1-one

The titled compound was prepared as colorless oil by procedures similar to the procedures of Example 1-(2), using 1-(4-trifluoromethoxyphenyl)piperazine. Yield 93%.

¹H NMR (CDCl₃, 400 MHz) δ: 2.25 (s, 3H), 2.7-2.8 (m, 4H), 3.0-3.1 (m, 4H), 3.35 (brs, 4H), 3.78 (s, 2H), 6.76 (d, 1H, J=8Hz), 6.85 (d, 2H, J=8Hz), 7.09 (d, 2H, J=8Hz), 7.65 (d, 2H, J=8Hz), 7.73 (dd, 1H, J=2, 8Hz), 7.78 (d, 1H, J=2Hz), 8.00 (d, 2H, J=8Hz).

### (2) Ethyl 2-[4-[3-[4-[4-(4-trifluoromethoxyphenyl)-piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 85%.

¹H NMR (CDCl₃, 400 MHz) δ: 1.20 (t, 3H, J=7Hz), 1.64 (s, 6H), 2.23 (s, 3H), 2.6-2.8 (m, 4H), 3.1-3.2 (m, 4H), 3.35 (brs, 4H), 3.78 (s, 2H), 4.20 (q, 2H, J=7Hz), 6.60 (d, 1H, J=8Hz), 6.85 (d, 2H, J=8Hz), 7.09 (d, 2H, J=8Hz), 7.65 (d, 2H, J=8Hz), 7.70 (dd, 1H, J=2, 8Hz), 7.78 (d, 1H, J=2Hz), 8.00 (d, 2H, J=8Hz).

### (3) 2-[4-[3-[4-[4-(4-Trifluoromethoxyphenyl)piperazin-1-ylmethyl]-2-(4-trifluoromethylphenyl)-5-thiazolyl]propionyl]-2-methylphenoxy]-2-methylpropionic acid

The titled compound was prepared as a pale yellow amorphous product by procedures similar to the procedures of Example 1-(4). Yield 89%.

¹H NMR (CD₃OD, 400 MHz) δ: 1.60 (s, 6H), 2.22 (s, 3H), 3.0-3.5 (m, 12H), 4.15 (s, 2H), 6.81 (d, 1H, J=8Hz), 7.0-7.2 (m, 4H), 7.7-7.9 (m, 4H), 8.10 (d, 2H, J=8Hz).

### [Example 16] 2-[4-[3-[4-[4-(4-Isopropyloxyphenyl)piperazin-1-ylmethyl]-2-(4-trifluoromethylphenyl)-5-thiazolyl]propionyl]-2-methylphenoxy]-2-methylpropionic acid

### (1) 1-(4-Hydroxy-3-methylphenyl)-3-[4-[4-(4-isopropyloxyphenyl)piperazin-1-ylmethyl]-2-(4-trifluoromethylphenyl)thiazol-5-yl]propan-1-one

The titled compound was prepared as colorless oil by procedures similar to the procedures of Example 1-(2) using 1-(4-isopropyloxyphenyl)piperazine. Yield 93%.

¹H NMR (CDCl₃, 400 MHz) δ: 1.29 (d, 6H, J=6Hz), 2.25 (s, 3H), 2.7-2.8 (m, 4H), 3.0-3.1 (m, 4H), 3.35 (brs, 4H), 3.78 (s, 2H), 4.40 (dq, 1H, J=6Hz, J=6Hz), 6.7-6.9 (m, 5H), 7.65 (d, 2H, J=8Hz), 7.73 (dd, 1H, J=2, 8Hz), 7.78 (d, 1H, J=2Hz), 8.00 (d, 2H, J=8Hz).

### (2) Ethyl 2-[4-[3-[4-[4-(4-isopropyloxyphenyl)piperazin-1-ylmethyl]-2-(4-trifluoromethylphenyl)-5-thiazolyl]propionyl]-2-methylphenoxy]-2-methylpropionate

The titled compound was prepared as colorless oil by procedures similar to the procedures of Example 3-(1). Yield 85%.

¹H NMR (CDCl₃, 400 MHz) δ: 1.20 (t, 3H, J=7Hz), 1.29 (d, 6H, J=6Hz), 1.64 (s, 6H), 2.25 (s, 3H), 2.7-2.8 (m, 4H), 3.0-3.1 (m, 4H), 3.35 (brs, 4H), 3.78 (s, 2H), 4.17 (q, 1H, J=7Hz), 4.42 (dq, 1H, J=6Hz, J=6Hz), 6.60 (d, 1H, J=8Hz), 6.7-6.8 (m, 4H), 7.65 (d, 2H, J=8Hz), 7.71 (dd, 1H, J=2, 8Hz), 7.79 (d, 1H, J=2Hz), 8.00 (d, 2H, J=8Hz).

### (3) 2-[4-[3-[4-[4-(4-Isopropyloxyphenyl)piperazin-1-ylmethyl]-2-(4-trifluoromethylphenyl)-5-thiazolyl]propionyl]-2-methylphenoxy]-2-methylpropionic acid

The titled compound was prepared as a pale yellow amorphous product by procedures similar to the procedures of Example 1-(4). Yield 89%.

¹H NMR (CD₃OD, 400 MHz) δ: 1.26 (d, 6H, J=6Hz), 1.60 (s, 6H), 2.23 (s, 3H), 3.2-3.5 (m, 12H), 4.25 (s, 2H), 4.47 (dq, 1H, J=6Hz, J=6Hz), 6.8-7.0 (m, 5H), 7.7-7.8 (m, 4H), 8.10 (d, 2H, J=8Hz).

### [Example 17] Pharmacological Experimental

### I. Procedures of Experimental

The PPAR activating effects of test compounds (compounds of Examples) were measured by the following method:

A receptor expression plasmid (pSG5-GAL4-hPPAR α or γ or δ (LBD)), a luciferase expression plasmid (pUC8-MH100x4-TK-Luc) and β-galactosidase expression plasmid (pCMX-β-GAL)(Kliewer, S.A., et. al.,(1992) Nature, 358:771-774) are transfected into CV-1 cells (ATCC).
After gene transfer utilizing a lipofection reagent DMRIE-C or Lipofectamin 2000 (Invitrogen), it is incubated for approx. 40 hours in the presence of the test compound. Then, the luciferase activity and β-GAL activity are measured on the soluble cells. The luciferase activity is calibrated by the β-GAL activity. A relative ligand activity is calculated for each of the PPAR α, γ and δ under the following conditions: a relative activity of PPAR α is calculated in consideration of a luciferase activity (assigned to 100%) of cells treated with GW-590735 (PPARα-selective agonist); a relative activity of PPAR γ is calculated in consideration of a luciferase activity (assigned to 100%) cells treated with Rosiglitazone; and a relative activity of PPAR δ is calculated in consideration of a luciferase activity (assigned to 100%) of cells treated with GW-501516. Then, EC₅₀ was obtained.

### II. Experimental Results

Experimental Results are set forth in Table 17.

**Table 17**

| Test compound | α | γ | δ |
|---|---|---|---|
| Example 3 | >10 | 0.66 | 0.92 |
| Example 4 | 0.21 | 1.3 | 4.4 |
| Example 5 | 0.090 | 3.2 | 5.2 |
| Example 6 | 0.55 | 0.49 | 4.9 |
| Example 11 | 0.092 | 0.30 | 3.0 |
| Example 12 | 0.94 | 1.7 | 3.8 |
| Example 13 | 0.33 | 0.28 | 1.1 |
| Example 15 | 0.29 | 0.75 | 6.3 |
| Example 16 | 0.18 | 0.29 | 2.3 |
| Rosiglitazone | >10 | 0.10 | >10 |
| KRP-297 | 0.40 | 0.90 | 13.9 |

| | | | |
|---|---|---|---|
| PPAR activity: EC₅₀ (µM), relative value (%) of the test compound (10⁻⁶M) to 100% of the control compound α: GW-590735 - 10⁻⁶ M γ: Rosiglitazone - 10⁻⁵ M δ: GW-501516 - 10⁻⁷ M | | | |

As is clear from Table 17, the compounds of Examples show excellent PPAR-activating effect. Particularly, the compound of Example 5 shows strong and selective PPARα agonist effect, and the compounds of Examples 11 and 16 show good α/γ dual agonist effects.

## Claims

1. A compound having the following formula (I) or a salt thereof: 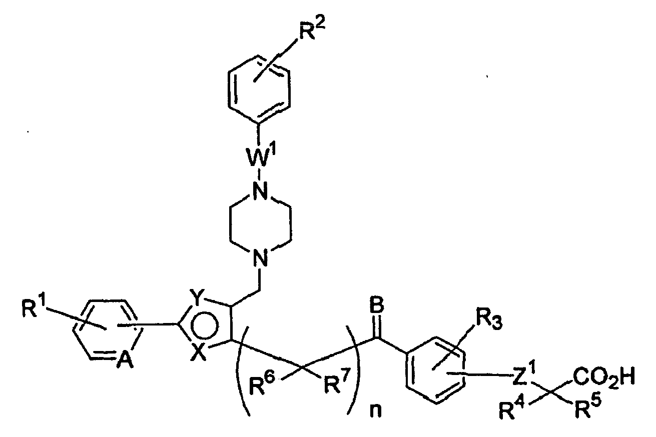 in which
A represents CH or a nitrogen atom;
B represents an oxygen atom or C(R⁸) (R⁹) in which each of R⁸ and R⁹ independently represents a hydrogen atom or an alkyl group having 1 to 8 carbon atoms;
W¹ represents a bond, C(=O), or (-C(R¹⁰) (R¹¹)-)ₘ in which each of R¹⁰ and R¹¹ independently a hydrogen or an alkyl group having 1 to 8 carbon atoms and m represents an integer of 1 to 3;
X and Y differ from each other, and each represents an oxygen atom, a sulfur atom, a nitrogen atom, or CR¹² in which R¹² represents a hydrogen atom or an alkyl group having 1 to 8 carbon atoms;
Z¹ represents a bond, an oxygen atom, a sulfur atom, or C(R¹³) (R¹⁴) in which each of R¹³ and R¹⁴ independently represents a hydrogen atom or an alkyl group having 1 to 8 carbon atoms;
each of R¹, R² and R³ independently represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms which is substituted with a halogen atom, an alkoxy group having 1 to 8 carbon atoms which is substituted with a halogen atom, hydroxyl, nitro, an acyl group having 2 to 8 carbon atoms, an aryl group having 6 to 10 carbon atoms, or a 5- or 6-membered heterocyclic group;
each of R⁴ and R⁵ independently represents a hydrogen atom, an alkyl group having 1 to 8 carbon atoms, or an alkyl group having 1 to 8 carbon atoms which is substituted with a halogen atom;
each of R⁶ and R⁷ independently represents a hydrogen atom, an alkyl group having 1 to 8 carbon atoms, an alkenyl group having 2 to 8 carbon atoms, an alkynyl group having 2 to 8 carbon atoms, or an alkyl group having 1 to 8 carbon atoms which is substituted with a halogen atom; and
n represents an integer of 1 to 5.

2. The compound or a salt thereof according to claim 1, wherein A is CH.

3. The compound of a salt thereof according to claim 1 or 2, wherein B is an oxygen atom.

4. The compound of a salt thereof according to any one of claims 1 to 3, wherein W¹ is a bond.

5. The compound of a salt thereof according to any one of claims 1 to 3, wherein W¹ is methylene or C(C=O).

6. The compound of a salt thereof according to any one of claims 1 to 5, wherein X and Y differ from each other, and each is an oxygen atom, a sulfur atom, or a nitrogen atom.

7. The compound of a salt thereof according to any one of claims 1 to 5, wherein X is a sulfur atom, and Y is a nitrogen atom.

8. The compound of a salt thereof according to any one of claims 1 to 7, wherein Z¹ is an oxygen atom or a sulfur atom.

9. The compound of a salt thereof according to any one of claims 1 to 8, wherein each of R¹, R² and R³ independently is a hydrogen atom, an alkyl group having 1 to 8 carbon atoms, an alkenyl group having 2 to 8 carbon atoms, an alkoxy group having 1 to 8 carbon atoms, a halogen atom, an alkyl group having 1 to 8 carbon atoms which is substituted with a halogen atom, or an alkoxy group having 1 to 8 carbon atoms which is substituted with a halogen atom.

10. The compound of a salt thereof according to any one of claims 1 to 9, wherein each of R⁴ and R⁵ independently is a hydrogen atom or methyl.

11. The compound of a salt thereof according to any one of claims 1 to 10, wherein each of R⁶ and R⁷ independently is a hydrogen atom or an alkyl group having 1 to 8 carbon atoms.

12. The compound of a salt thereof according to any one of claims 1 to 11, wherein n is an integer of 2 to 4.

13. The compound of a salt thereof according to any one of claims 1 to 11, wherein n is 2.

14. A compound having the following formula (II) or a salt thereof: 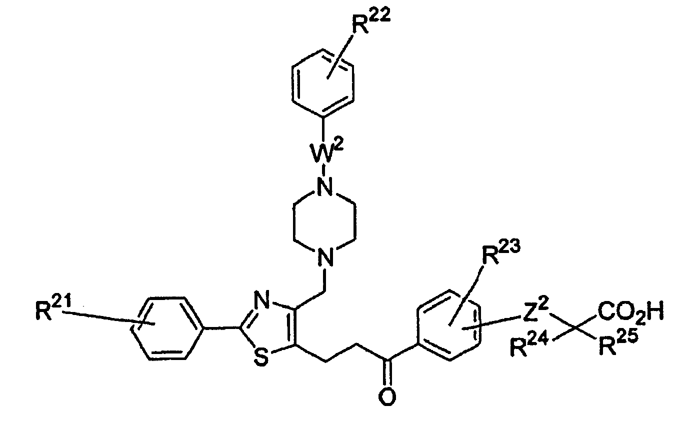 in which
W² represents a bond, C(=O), or -CH₂;
Z² represents an oxygen atom or a sulfur atom;
each of R²¹, R²² and R²³ independently represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms which is substituted with a halogen atom, an alkoxy group having 1 to 8 carbon atoms which is substituted with a halogen atom, hydroxyl, nitro, an acyl group having 2 to 8 carbon atoms, an aryl group having 6 to 10 carbon atoms, or a 5- or 6-membered heterocyclic group;
each of R²⁴ and R²⁵ independently represents a hydrogen atom, an alkyl group having 1 to 8 carbon atoms, or an alkyl group having 1 to 8 carbon atoms which is substituted with a halogen atom.

15. The compound of a salt thereof according to claim 14, wherein W² is a bond.

16. The compound of a salt thereof according to claim 14 or 15, wherein each of R²¹, R²² and R²³ independently is a hydrogen atom, an alkyl group having 1 to 8 carbon atoms, an alkenyl group having 2 to 8 carbon atoms, an alkoxy group having 1 to 8 carbon atoms, a halogen atom, an alkyl group having 1 to 8 carbon atoms which is substituted with a halogen atom, or an alkoxy group having 1 to 8 carbon atoms which is substituted with a halogen atom.

17. The compound of a salt thereof according to any one of claims 14 to 16, wherein each of R²⁴ and R²⁵ independently is a hydrogen atom or methyl.

18. An activator for peroxisome proliferator activated receptor containing a compound or a salt thereof according to any one of claims 1 to 17 as an effective component.
